(19) Europäisches Patentamt
European Patent Office
Office européen des brevets

(11) Veröffentlichungsnummer: **0 005 779**
**B1**

(12) # EUROPÄISCHE PATENTSCHRIFT

(21) Anmeldenummer: **79101537.3**

(22) Anmeldetag: **21.05.79**

(51) Int. Cl.$^3$: **C 07 C 59/54**, C 07 B 3/00, C 07 D 317/60

(54) **Verfahren zur Herstellung von Arylglyoxylsäuren**

(30) Priorität: **03.06.78 DE 2824407**

(43) Veröffentlichungstag der Anmeldung: **12.12.79 Patentblatt 79/25**

(45) Bekanntmachung des Hinweises auf die Patenterteilung: **26.11.80 Patentblatt 80/24**

(84) Benannte Vertragsstaaten: **BE CH DE FR GB IT NL**

(56) Entgegenhaltungen:
**CHEMICAL ABSTRACTS, Vol. 87, Nr. 25, 19.Dezember 1977, Columbus, Ohio, USA, I.V. KOZHEVNIKOV et al.: "Liquid-phase oxidation of alcohols catalyzed by a palladium (II) - heteropoly acid system", Seite 661, Zusammenfassung Nr. 200733z**

(73) Patentinhaber: **BAYER AG**
**Zentralbereich Patente, Marken und Lizenzen**
**D - 5090 Leverkusen 1, Bayerwerk (DE)**

(72) Erfinder: **Fiege, Helmut, Dr.**
**Walter-Flex-Strasse 8**
**D - 5090 Leverkusen 1 (DE)**
**Wedemeyer, Karlfried, Dr.**
**Bilharzstrasse 7**
**D - 5000 Köln 80 (DE)**
**Bauer, Kurt, Dr.**
**Corveyblick 41**
**D - 3450 Holzminden (DE)**
**Mölleken, Reiner, Dr.**
**Forstbachtal 35**
**D - 3451 Golmbach Ortsteil Warbsen (DE)**

**EP 0 005 779 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Verfahren zur Herstellung von Arylglyoxylsäuren

Die vorliegende Erfindung betrifft ein Verfahren zur Herstellung von Arylglyoxylsäuren durch Oxidation von $\alpha$-Hydroxyarylessigsäuren (Arylglykolsäuren) mit Sauerstoff oder molekularen Sauerstoff enthaltenden Gasen in wäßrigalkalischen Medien, die gegebenenfalls organische Lösungsmittel enthalten können, bei Temperaturen bis zum Siedepunkt des Reaktionsgemisches in Gegenwart von Platinmetall-Katalysatoren. Arylglyoxylsäuren sind wertvolle Zwischenprodukte in der organischen Synthese, z.B. bei der Herstellung von Pflanzenschutzmitteln, von Riechund Geschmacksstoffen sowie von Arzneimitteln.

Es ist bekannt, daß man $\alpha$-Hydroxyarylessigsäuren zu Arylglyoxylsäuren, insbesondere Mandelsäure zu Phenylglyoxylsäure, oxidieren kann. Diese Oxidationen erfordern jedoch relative teure, umweltbelastende Oxidationsmittel (wie Kaliumpermanganat, Chrom(VI)-Verbindungen, Osmium(VIII)-Verbindungen, Salpetersäure) sowie Betriebsbedingungen, die technisch wenig attraktiv sind. Gewöhnlich wird mit Kaliumpermanganat in wäßrig-alkalischem Medium oxidiert. Dabei läßt es sich selbst unter günstigsten Bedingungen (Eiskühlung, Mandelsäurekonzentrationen unter 10 Gew.-%) nicht verhindern, daß ein beträchtlicher Teil des Ausgangsmaterials durch Überoxidation (z.B. zu Benzoesäure) verlorengeht und die Ausbeuten dementsprechend unbefriedigend sind (vgl. B.B. CORSON et al, Organic Synthesis, Coll. Vol. I, 2. Auflage (1956), Seite 241—245; hiernach werden Ausbeuten von lediglich 50—67% erzielt).

Bei der Oxidation von Mandelsäure in wäßriger Lösung mit reinem Sauerstoff bei Zutritt von Sonnenlicht werden als Reaktionsprodukte keine Phenylglyoxylsäure, sondern nur Benzaldehyd, Salicylaldehyd, Benzoesäure und etwas Salicylsäure erhalten (vgl. G. CIAMICIAN et al., Ber. dtsch. chem. Ges. *46,* Seite 1559 (1913).

Es wurde nun gefunden, daß man Arylglyoxylsäuren in sehr hoher Ausbeute und ausgezeichneter Reinheit erhält, wenn man $\alpha$-Hydroxy-arylessigsäuren der Formel

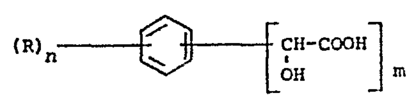

(I)

in der

m für 1, 2 oder 3 steht,

n für die Zahl, die sich aus der Differenz (6-m) ergibt, steht und die Reste

R entweder einzeln und unabhängig voneinander für Wasserstoff, Alkyl, Cycloalkyl, Aryl, Aralkyl, Alkoxy, Cycloalkoxy, Aryloxy, Hydroxy, Halogen, Aminoalkyl und/oder Carboxy stehen, oder in der zwei Reste R gemischen für die Methylendioxygruppe oder einen anderen anellierten, gegebenenfalls heterocyclischen und gegebenenfalls substituierten Ring stehen,

mit Sauerstoff oder molekularen Sauerstoff enthaltenden Gasen in wäßrigalkalischem Medium in Gegenwart von Platinmetall-Katalysatoren bei gleichzeitiger Anwesenheit von Blei und/oder Wismut und/oder deren Verbindungen als Aktivatoren und gegebenenfalls in Gegenwart eines inerten organischen Lösungsmittels bei Temperaturen bis zum Siedepunkt des Reaktionsgemisches oxidiert.

Die $\alpha$-Hydroxyaryl-essigsäuren (I) können in der D-, der L- oder in der D,L-Form vorliegen. Auch Gemische verschiedener $\alpha$-Hydroxyaryl-essigsäuren können zur Oxidation eingesetzt werden.

Falls die Ausgangsverbindungen der Formel (I) mehrere $\alpha$-Hydroxy-essigsäuregruppierungen aufweisen (m = 2 oder 3), läßt sich die Oxidation auch so leiten, daß von den verschiedenen $\alpha$-Hydroxyessigsäuregruppen nur eine oder zwei selektiv zur Glyoxylsäuregruppe oxidiert werden. Auf diese Weise werden Arylglyoxylsäuren erhalten, die eine oder zwei $\alpha$-Hydroxy-essigsäuregruppen besitzen.

Das erfindungsgemäße Verfahren weist eine Reihe von Vorteilen auf. So wird als Oxidationsmittel Sauerstoff verwendet, der allgemein zur Verfügung steht, billig ist und nicht zu umweltbelastenden Nebenprodukten führt. Von besonderer Bedeutung ist, daß die Oxidation hochselektiv verläuft, kaum zu Überoxidationen führt und damit wesentlich höhere Ausbeuten und reinere Produkte liefert als die bekannten Verfahren; dies bedeutet zugleich, daß eine Vergeudung wertvoller Rohstoffe vermieden wird. Ein weiterer wichtiger Vorteil besteht schließlich darin, daß die technische Durchführung wesentlich erleichtert ist, weil die höheren Reaktionstemperaturen die Wärmeabfuhr erleichtern, die höheren Arylglyoxylsäure-Konzentrationen höhere Raum-Zeit-Ausbeuten und verteilhaftere Aufarbeitung ermöglichen, die Oxidation über die Sauerstoffaufnahme gut gesteuert werden kann und die Abtrennung und Beseitigung von problematischen Oxidationsmittel-Folgeprodukten entfällt.

Es ist im Hinbick auf den Stand der Technik als ausgesprochen überraschend zu bezeichnen, daß es unter den Bedingungen des erfindungsgemäßen Verfahrens gelingt, $\alpha$-Hydroxy-arylessigsäuren, wie z.B. Mandelsäure, auf technisch einfache Weise mit sehr hohen Ausbeuten in sehr reine Arylglyoxylsäuren, wie z.B. Phenylglyoxylsäure, zu überführen. Besonders überraschend ist es, daß im Gegensatz zu den vorbekannten Verfahren die Oxidation hochselektiv ist und daß keine wesentliche Überoxidation, z.B. zu Benzoesäuren, eintritt.

Bei Verwendung von 3,4-Methylendioxy-mandelsäure als Ausgangswerbindung kann der Reaktionsablauf durch das folgende Formelschema wiedergegeben werden:

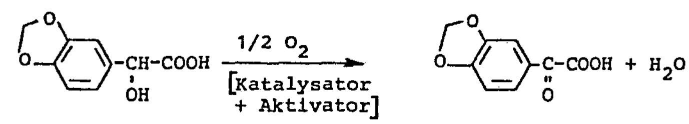


Die erfindungsgemäß als Ausgangsstoffe zu verwendenden $\alpha$-Hydroxy-arylessigsäuren sind durch die Formel (I) allgemein definiert. In dieser Formel steht m vorzugsweise für die Zahl 1; die Reste R stehen einzeln und unabhängig voneinander vorzugsweise für Wasserstoff, Alkyl mit 1 bis 12 C-Atomen, Cycloalkyl mit 3 bis 6 C-Atomen, Phenyl, Benzyl, Alkoxy mit 1 bis 12 C-Atomen, Cycloalkoxy mit 3 bis 6 C-Atomen, Phenoxy, Hydroxy, Fluor, Chlor, Brom, Jod, Aminoalkyl mit 1 bis 4 C-Atomen und/oder die Carboxy-Gruppe; zwei Reste R stehen gemeinsam vorzugsweise für die Methylendioxy-gruppe.

Besonders bevorzugt sind diejenigen $\alpha$-Hydroxyarylessigsäuren der Formel (I), in denen m für 1 und die Reste R für Wasserstoff stehen (d.h. unsubstituierte Mandelsäure), ferner solche Verbindungen, in denen m für 1 und entweder bis zu drei Reste R einzen und unabhängig voneinander für Alkyl mit 1 bis 6 C-Atomen (insbesondere Methyl, Athyl, Propyl, Isopropyl, Butyl, Pentyl und Hexyl), für Phenyl, Benzyl, Methoxy, Äthoxy, Isopropoxy, Phenoxy, Hydroxy, Carboxy, Fluor, Chlor und/oder Brom stehen oder zwei Reste R gemeinsam für die Methylendioxy-Gruppe stehen, wobei diese Reste R in 3-, 4- und/oder 5-Stellung stehen, (d.h. bestimmte einfach, zweifach und dreifach meta- und/oder parasubstituierte Mandelsäuren). Im einzelnen seien folgende Verbindungen der Formel (I) beispielhaft genannt:

Mandelsäure
3-Methyl-mandelsäure
4-Methyl-mandelsäure
3,4-Dimethyl-mandelsäure
3,5-Dimethyl-mandelsäure
3,4,5-Trimethyl-mandelsäure
3-Äthyl-mandelsäure
4-Äthyl-mandelsäure
3,4-Diäthyl-mandelsäure
3,5-Diäthyl-mandelsäure
3,4,5-Triäthyl-mandelsäure
3-n-Propyl-mandelsäure
4-n-Propyl-mandelsäure
3,4-Di-n-propyl-mandelsäure
3,4,5-Tri-n-propyl-mandelsäure
3-i-Propyl-mandelsäure
4-i-Propyl-mandelsäure
3,4-Di-isopropyl-mandelsäure
3,5-Di-isopropyl-mandelsäure
3-n-Butyl-mandelsäure
4-n-Butyl-mandelsäure
3,4-Di-n-butyl-mandelsäure
3,5-Di-n-butyl-mandelsäure
3-sek.-Butyl-mandelsäure
4-sek.-Butyl-mandelsäure
4-tert.-Butyl-mandelsäure
3-Hydroxy-mandelsäure
4-Hydroxy-mandelsäure
3-Hydroxy-4-methyl-mandelsäure
4-Hydroxy-3-methyl-mandelsäure
4-Hydroxy-3,5-dimethyl-mandelsäure
4-Hydroxy-5-isopropyl-mandelsäure
4-Hydroxy-3-methyl-5-isopropyl-mandelsäure
4-Hydroxy-5-tert.-butyl-mandelsäure
4-Hydroxy-3,5-di-tert.-butyl-mandelsäure
3,5-Dihydroxy-mandelsäure
4-Hydroxy-3-methoxy-mandelsäure
4-Hydroxy-3-äthoxy-mandelsäure
3-Hydroxy-4-methoxy-mandelsäure

3,4-Dimethoxy-mandelsäure
3,4-Methylendioxy-mandelsäure
4-Hydroxy-3,5-dimethoxy-mandelsäure
3-Phenoxy-mandelsäure
4-Phenoxy-mandelsäure
3-Methoxy-mandelsäure
4-methoxy-mandelsäure
3,5-Dimethoxy-mandelsäure
3-Hydroxy-4-isopropoxy-mandelsäure
4-Hydroxy-3-isopropoxy-mandelsäure
4-Hydroxy-3-chlor-mandelsäure
3-Hydroxy-4-chlor-mandelsäure
3-Chlormandelsäure
4-Chlormandelsäure
4-Benzylmandelsäure
3,4-Dichlormandelsäure
3,5-Dichlormandelsäure
3,4,5-Trichlormandelsäure
3-Brom-mandelsäure
4-Brom-mandelsäure
3,4-Dibrom-mandelsäure
3,5-Dibrom-mandelsäure
3-Fluor-mandelsäure
4-Fluor-mandelsäure

$\alpha$-Hydroxyarylessigsäuren der Formel (I) sind an sich bekannt oder können nach bekannten Verfahren hergestellt werden, beispielsweise durch Addition von Blausäure an entsprechend substituierte Benzaldehyde und anschließende Hydrolyse der Benzaldehyd-cyanhydrine oder durch Verseifung von 2,2-Dihalogenacetophenonen (vgl. J. G. ASTON et al., Org. Synth. Coll. Vol. III, 1955, S. 538—541; dort, sowie in Rodd's Chemistry of Carbon Compounds Vol. III, Part E, 2. Auflage (1974), S. 105—109, finden sich weitere Synthesehinweise).

Unter "wäßrig-alkalischem Medium" ist zu verstehen, daß die $\alpha$-Hydroxyarylessigsäuren bei der Oxidation durch Umsetzen mit Alkali im wäßrigen Medium ganz oder teilweise als Alkalisalze vorliegen. Das Alkali kann dabei so bemessen sein, daß das Reaktionsgemisch alkalisch reagiert, also einen pH-Wert > 7 aufweist. Vorteilhaft wird das Alkali in solchen Mengen angewendet, daß auf 1 Mol Carboxylgruppe in der $\alpha$-Hydroxyarylessigsäure 0,3 bis 5, vorzugsweise 0,5 bis 2,5 Äquivalente Alkali kommen. Insbesondere bevorzugt werden etwa 0,9 bis 1,8 Äquivalent Alkali je Mol Carboxylgruppe in der $\alpha$-Hydroxyarylessigsäure angewendet.

Das Alkali kann zur Lösung bzw. Suspension der $\alpha$-Hydroxyarylessigsäure in Wasser gegeben werden, oder die $\alpha$-Hydroxyarylessigsäure kann in der Alkali-Lösung gelöst bzw. suspendiert werden.

Als Alkali werden bevorzugt Hydroxide oder Carbonate des Natriums eingesetzt.

Die Konzentration der $\alpha$-Hydroxyarylessigsäure in der wäßrig-alkalischen Reaktionslösung wird im allgemeinen so gewählt, daß sowohl die $\alpha$-Hydroxyarylessigsäure als auch die gebildete Arylglyoxylsäure während der Reaktion gelöst vorliegen. Bewährt haben sich Konzentrationen von 5 bis 40 Gew.-% $\alpha$-Hydroxyarylessigsäure. Es können allerdings auch Suspensionen oxidiert werden. Auch lassen sich Gemische verschiedener $\alpha$-Hydroxyarylessigsäure oxidieren.

Werden im erfindungsgemäßen Verfahren als Ausgangsverbindungen $\alpha$-Hydroxyarylessigsäure eingesetzt, die im wäßrig-alkalischen Reaktionsmedium schwer- bzw. unlöslich sind und/oder sind die entstehenden Arylglyoxylsäuren schwer bzw. unlöslich in der wäßrig-alkalischen Lösung, so kann die Oxidation evtl. gestört werden. Solche Störungen lassen sich in der Regel jedoch beheben, wenn man die Reaktion in Gegenwart eines geeigneten Lösungsmittels für die schwer- bzw. unlösliche Verbindung durchführt.

Ein solches Lösungsmittel kann mit dem wäßrig-alkalischen Reaktionsmedium vollständig, partiell oder auch nicht mischbar sein, Wesentlich ist, daß das Lösungsmittel unter den Reaktionsbedingungen inert ist. Welche Lösungsmittel und welche Lösungsmittelmenge im Einzelfall anzuwenden sind, kann durch Vorversuche leicht ermittelt werden. Als Lösungsmittel kommen sowohl aprotische Lösungsmittel, also Kohlenwasserstoffe wie Benzol und Hexan, Äther wie Dioxan oder Ketone wie Aceton, und protische Lösungsmittel, insbesondere Alkohole wie z.B. tert.-Butanol in Betracht.

Unter den erfindungsgemäßen Bedingungen kann eine Oxidationswirkung bei alle Temperaturen beobachtet werden, bei denen eine flüssige wäßrige Phase vorliegt. Dementsprechend reicht die mögliche Reaktionstemperatur vom Erstarrungspunkt bis zum Siedepunkt des Reactionsgemisches. Die im Einzelfall anzuwendende Reaktionstemperatur richtet sich u.a. nach den Substanzeigenschaften (wie Löslichkeit, Stabilität der Edukte und Produkte) und technischen Gegebenheiten (gewünschte Reaktionsgeschwindigkeit, Wärmeabfuhr). Bevorzugt wird im Temperaturbereich von 0° bis etwa 100°C gearbeitet.

Unter "Platinmetallen" die in dem erfindungsgemäßen Verfahren als Katalysatoren eingesetzt werden, sind die chemisch nahe verwandten, in der Natur meist gemeinsam auftretenden Metalle Platin, Palladium, Rhodium, Iridium, Ruthenium und Osmium zu verstehen. Bevorzugt werden die Platinmetalle Platin und Palladium eingesetzt, insbesondere Platin.

Die als Katalysatoren verwendeten Platinmetalle können den Reaktionskomponenten in verschiedenster Form zugegeben werden, beispielsweise in elementarer, d.h. metallischer, Form, z.B. als sogenanntes Mohr, in Kombination mit anderen Platinmetallen oder in Form von Verbindungen, z.B. als Oxide oder auch in Form anderer Verbindungen.

Die Platinmetalle können auch auf Träger aufgebracht sein. Als Träger geeignet sind beispielsweise Aktivkohle, Graphit, Kieselgur, Kieselgel, Spinelle, Aluminiumoxid, Asbest, Calciumcarbonat, Magnesiumcarbonat, Bariumsulfat oder auch organische Trägermaterialien. Besonders bewährt haben sich Aktivkohlen, beispielsweise aus Holz hergestellte billige pulverförmige Aktivkohlen, die vielfach für Entfärbungswecke verwendet werden.

Der Platinmetall-Gehalt dieser Trägerkatalysatoren kann in weiten Grenzen schwanken. Besonders bewährt haben sich Trägerkatalysatoren mit einem Platinmetall-Gehalt unter 10 Gew.-%, insbesondere solche mit Gehalten von 0,1 bis 2,5 Gew-% Platinmetall.

Die Mengen, in denen die Platinmetall-Katalysatoren verwendet werden, können in weiten Grenzen schwanken. Die Mengen hängen von der gewünschten Oxidationsgeschwindigkeit, der Art der zu oxidierenden $\alpha$-Hydroxyarylessigsäure, der Katalysatorform, der Aktivator-Art und -Menge usw. ab und lassen sich im speziellen Fall durch Vorversuche leicht ermitteln. So kann bei der Oxidation von $\alpha$-Hydroxyphenylessigsäure in Gegenwart von Bleiverbindungen als Aktivator schon innerhalb von 15 Minuten eine Phenylglyoxylsäure-Ausbeute von 99% d.Th. bei Anwesenheit von nur 75 mg Platin je Mol $\alpha$-Hydroxyphenylessigsäure erzielt werden. Bei Inkaufnahme längerer Oxidationszeiten kann auch in Gegenwart noch geringerer Platinmengen, z.B. 30 mg Platin je Mol $\alpha$-Hydroxyphenylessigsäure gearbeitet werden. Die Oxidation dauert in diesem Fall 70 Minuten, die Ausbeute beträgt ebenfalls rund 99% d.Th.

Im allgemeinen liegt die je Mol $\alpha$-Hydroxyessigsäuregruppe erforderliche Platinmetall-Menge unter 500 mg, in den meisten Fällen werden mit 10 bis 150 mg Platinmenge je Mol zu oxidierende $\alpha$-Hydroxyessigsäuregruppe ausreichend hohe Reaktionsgeschwindigkeiten erreicht.

Da bei Verwendung der erfindungsgemäßen Aktivatoren eine Teerbildung fast vollständig vermieden wird, können die Katalysatoren wiederholt eingesetzt werden. Durch diese Wiederverwendung kann der Verbrauch an Platinmetall-Katalysator je Mol $\alpha$-Hydroxyessigsäuregruppe auf 3 mg und darunter gesenkt werden, bevor eine Wiederaufarbeitung des Platinmetall-Katalysators erforderlich wird.

Als Aktivatoren haben sich vor allem Blei und/oder Wismut bewährt. Die Mengen, in denen die erfindungsgemäß zu verwendenden Aktivatoren eingesetzt werden, können in weiten Grenzen schwanken. Die Aktivatorwirkung macht sich bereits bei Zusätzen von $1 \times 10^{-6}$ Mol Mteall bzw. Metallverbindung je Mol $\alpha$-Hydroxyessigsäuregruppe deutlich bemerkbar. Es können auch 0,1 Mol oder mehr Aktivator je Mol $\alpha$-Hydroxyessigsäuregruppe eingesetzt werden, jedoch bieten diese hohen Zusätze im allgemeinen keinen Vorteil. Im allgemeinen haben sich Zusätze von $5 \times 10^{-6}$ Mol, vorzugsweise $1 \times 10^{-5}$ bis $1 \times 10^{-2}$ Mol Metall je Mol zu oxidierende $\alpha$-Hydroxyessigsäuregruppe bewährt.

Die erfindungsgemäß als Aktivatoren zu verwendenden Metalle können als solche, d.h. in elementarer Form und-oder in Form ihrer Verbindungen, z.B. als Oxide oder Salze von Wasserstoffsäuren wie Chloride, Bromide, Jodide, Sulfide, Selenide, Telluride, oder als Salze von anorganischen Sauerstoffsäuren wie Nitrate, Nitrite, Phosphite, Phosphate, Carbonate, Perchlorate, Antimonate, Arseniate, Selenite, Seleniate, Borate, oder als Salze von Sauerstoffsäuren, die von Übergangsmetallen abstammen, wie z.B. Vanadate, Niobate, Tantalate, Chromate, Molybdate, Wolframate, Permanganate, oder als Salze organischer aliphatischer oder aromatischer Säuren wie z.B. Formiate, Acetate, Propionate, Benzoate, Salicylate, Lactate, Mandelate, Glyoxylate, Arylglyoxylate, Citrate oder als Phenolate usw. eingesetzt werden. Die Aktivatoren können im Reationsgemisch jeweils löslich, teilweise löslich oder unlöslich sein.

Auch Kombinationen dieser Aktivatoren untereinander und/oder mit anderen, nicht als Aktivator beanspruchten Elementen oder Verbindungen können verwendet werden. Die erfindungsgemäßen Aktivatoren können in unterschiedlichen und auch gemischten Wertigkeitsstufen vorliegen; auch können Änderungen in der Wertigkeit während der Reaktion eintreten. Sofern die Aktivatoren nicht bereits als Oxide und/oder Hydroxyde zugegeben werden, ist es möglich, daß die sich im alkalischen Medium ganz oder teilweise in diese umwandeln. Nach der Reaktion kann der Platinmetall-Katalysator mit dem schwer löslichen Aktivator abfiltriert und in weiteren Oxidationen wiederverwendet werden. Verluste an Platinmetall-Katalysatoren und/oder Aktivator sind gegebenenfalls zu ersetzen.

Der Aktivator kann als Feststoff, vorzugsweise in fein verteilter Form, oder in gelöster Form den Reaktionskomponenten zugesetzt werden. Man kann den Aktivator auch schon bei der Herstellung des Platinmetall-Katalysators zugeben oder den Platinmetall-Katalysator mit dem Aktivator imprägnieren. Der Aktivator kann auch als Trägermaterial für das Platinmetall dienen.

Besonders bewährt hat sich die Kombination von Platin mit Blei und/oder Wismut.

Das erfindungsgemäße Verfahren wird üblicherweise so durchgeführt, daß man Sauerstoff oder molekularen Sauerstoff enthaltende Gase wie Luft mit der das alkalische Mittel, den Platinmetall-Katalysator und den erfindungsgemäßen Aktivator enthaltenden Lösung der $\alpha$-Hydroxyarylessigsäure

in guten Kontakt bringt. Gewöhnlich arbeitet man bei Atmosphärendruck (1 bar), jedoch kann auch bei höheren oder niedrigeren Drucken oxidiert werden, beispielsweise im Bereich von 0,5 bis 10 bar. Der Verlauf der Reaktion kann über die aufgenommene Sauerstoffmenge verfolgt werden und wird abgebrochen, wenn die für die gewünschte Arylglyoxylsäure theoretisch erforderliche Sauerstoffmenge aufgenommen ist. Meistens hört die Sauerstoffaufnahme in diesem Stadium von selbst auf oder sie verlangsamt sich. Der Fortgang der Reaktion kann auch auf andere Weise, z.B. durch Bestimmung der verbrauchten $\alpha$-Hydroxyarylessigsäure oder der gebildeten Arylglyoxylsäure verfolgt werden.

Zur Aufarbeitung werden Platinmetall-Katalysator nebst ungelöstem Aktivator vom Reaktionsgemisch abgetrennt, beispielsweise durch Filtrieren. Aus der alkalischen Reaktionslösung werden die Arylglyoxylsäuren durch Ansäuern auf einen pH-Wert unterhalb von 6 freigesetzt und nach bekannten Verfahren wie Dekantieren, Abfiltrieren, Extrahieren und/oder durch Wasserdampfdestillation abgetrennt und erforderlichenfalls z.B. durch Umkristallisieren, Destillieren, Extrahieren oder Sublimieren weiter gereinigt.

Die Reihenfolge, in der Platinmetall-Katalysator, Aktivator, Alkali und $\alpha$-Hydroxyarylessigsäure zusammengegeben werden, ist beliebig. So können Platinmetall-Katalysator und Aktivator der wäßrig-alkalischen Hydroxyarylessigsäure-Lösung zugesetzt werden; man kann auch Platinmetall-Katalysator und Aktivator vorlegen und wäßrig-alkalische $\alpha$-Hydroxyarylessigsäure-Lösung zusetzen; schließlich ist es auch möglich, Platinmetall-Katalysator, einen Teil des wäßrigen Alkalis und den Aktivator vorzulegen und die $\alpha$-Hydroxyarylessigsäure zusammen mit dem restlichen Alkali zuzusetzen. Ferner ist es möglich, den Aktivator der Mischung der Reaktionskomponente zuzugeben.

Die nach dem erfindungsgemäßen Verfahren herstellbaren Arylglyoxylsäuren sind wichtige organische Zwischenprodukte und von großer Bedeutung. z.B. fur die Herstellung von Pflanzenschutzmitteln, von Reich- und Geschmacksstoffen sowie von Arzneimitteln.

So läßt such beispielsweise ausgehend von Phenylglyoxylsäure der herbizide Wirkstoff 3-Methyl-4-amino-6-phenyl-1,2,4-triazin-5 (4H)-on herstellen(vgl. DE—OS 2 224 161).

Das erfindungsgemäße Verfahren wird durch die nachfolgenden Herstellungsbeispiele veranschaulicht.

*Herstellungsbeispiele*

BEISPIEL 1

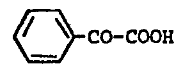

In einem mit Rührer, Thermometer und Gaszuleitung versehenen Reaktionsgefäß werden 0,75 g platinhaltige Aktivkohle (Platingehalt: 1 Gew.-%), 0,5 ml 0,1-molare $Pb(NO_3)_2$-Lösung (entsprechend einer Blei-Menge von $5 \times 10^{-5}$ Mol) und eine Lösung von 15,2 g (0,1 Mol) D,L-Mandelsäure in 100 ml 1,2 n Natronlauge eingebracht.

Nach Verdrängen der Luft aus dem Reaktionsgefäß durch Sauerstoff wird der Rührer angestellt und bei 70°C wird unter kräftigem Rühren reiner Sauerstoff unter Normaldruck in die Mischung eingeleitet. Nach 15 Minuten sind 0,05 Mol $O_2$ aufgenommen und die Sauerstoffaufnahme kommt zum Stillstand.

Nach Abfiltrieren des Kontaktes wird im Filtrat der Gehalt an Phenylgloxylsäure durch Differential-Puls-Polarographie bestimmt. Als Grundelektrolyt diente 1 n LiOH. Die Bestimmung erfolgte gegen Phenylgloxylsäure-Lösung bekannten Gehaltes, die bei einer Wiederholungsmessung als interner Standard zugegeben wurde. Die Bestimmung ergab eine Phenylgloxylsäure-Ausbeute von 99,3% d.Th.

Die Phenylgloxylsäure kann auch durch Ansäuern mit Schwefelsäure freigesetzt, z.B. mit Äther aus der Lösung extrahiert und nach Verdampfen des Äthers in freier Form erhalten werden. Der abfiltrierte Kontakt kann wiederverwendet werden.

BEISPIELE 2 BIS 5

Es wird wie in Beispiel 1 gearbeitet, jedoch mit dem Unterschied, daß die Mandelsäure- und die Natronlauge-Konzentration erhöht werden. Das Natronlauge-Volumen (100 ml), die Menge an platinhaltiger Aktivkohle (0,75 g mit 1 Gew.-% Pt), die Bleimenge ($5 \times 10^{-5}$ Mol als Bleinitrat), die Temperatur (70°C) und der Druck (1 bar) bleiben gleich.

In Tabelle 1 sind die Mandelsäure- und NaOH-Konzentrationen, die pro Mol Mandelsäure eingesetzten NaOH-, Platin- und Blei-Mengen, die erzielten Phenylglyoxylsäure-Ausbeuten und die zu ihrer Erlangung angewendeten Reaktionszeiten angegeben:

TABELLE 1

| Beispiel Nr. | Mandelsäure Mol | Normalität der NaOH (100 ml) | pro mol Mandelsäure eingesetzte Menge | | | | |
|---|---|---|---|---|---|---|---|
| | | | NaOH (Mol) | Platin (mg) | Blei (Mol) | Reaktionsdauer (Stdn.) | Ausbeute % d. Th. |
| 1 [a] | 0,1 | 1,2 | 1,20 | 75 | $5 \cdot 10^{-4}$ | 0,25 | 99,3 |
| 2 | 0,15 | 1,7 | 1,13 | 50 | $3,3 \cdot 10^{-4}$ | 0,43 | 99,1 |
| 3 | 0,20 | 2,2 | 1,10 | 37,5 | $2,5 \cdot 10^{-4}$ | 0,78 | 99,0 |
| 4 | 0,25 | 2,7 | 1,08 | 30 | $2 \cdot 10^{-4}$ | 1,13 | 98,5 |
| 5 | 0,30 | 3,2 | 1,07 | 25 | $1,7 \cdot 10^{-4}$ | 2,25 | 98,0 |

a) zum Vergleich mit in die Tabelle aufgenommen.

## BEISPIELE 6 BIS 10

Es wird wie in Beispiel 1 gearbeitet, jedoch mit dem Unterschied, daß 38 g (0,25 Mol) Mandelsäure, 100 ml 2,7 n NaOH und unterschiedliche Aktivatormengen (Blei (II)-nitrat) eingesetzt werden.

Die Menge an platinhaltiger Aktivkohle (0,75 g mit 1 Gew.-% Pt-Gehalt), die Temperatur (70°C) und der Druck (1 bar) bleiben gleich.

In Tabelle 2 sind die pro Mol Mandelsäure eingesetzten Bleimengen (in Mol), die Phenylgloxylsäure-Ausbeuten und die zu ihrer Erlangung angewendeten Reaktionszeiten angegeben.

TABELLE 2

| Beispiel Nr. | pro Mol Mandelsäure eingesetzte Menge Blei (Mol) | Reaktionsdauer (Stunden) | Phenylglyoxylsäure-Ausbeute (% d. Th.) |
|---|---|---|---|
| 6 | $2 \cdot 10^{-3}$ | 1,4 | 98,1 |
| 7 | $1 \cdot 10^{-3}$ | 1,1 | 98,9 |
| 8 | $1 \cdot 10^{-4}$ | 1,5 | 98 |
| 9 | $2 \cdot 10^{-5}$ | 2,5 a) | 86 a) |
| 10 c) | 0 | 2,5 b) | 0 |

a) Oxidation nach dieser Zeit vorzeitig abgebrochen
b) praktisch keine $O_2$-Aufnahme
c) Beispiel 10 ist ein *Vergleichsbeispiel,* das zeigt, daß ohne Aktivator-Zusatz praktisch keine Oxidation eintritt.

## BEISPIELE 11 BIS 13

Es wird wie im Beispiel 1 gearbeitet, jedoch mit dem Unterschied, daß 38 g (0,25 Mol) Mandelsäure, 100 ml 2,7 n NaOH, 2,5 x $10^{-4}$ Mol Pb $(NO_3)_2$ als 0,5-molare Lösung und verschiedene Reaktionstemperaturen angewendet werden.

Die menge an platinhaltiger Aktivkohle (0.75 g mit 1 Gew.-% Platin) und der $O_2$-Druck (1 bar) bleiben gleich.

In Tabelle 3 sind die in Abhängigkeit von der Temperatur erzielten Ausbeuten und die zu ihrer Erlangung angewendeten Reaktionszeiten angegeben:

TABELLE 3

| Versuch Nr. | Temperatur (°C) | Reaktionsdauer (Stunden) | Phenylglyoxylsäure-Ausbeute (% d. Th.) |
|---|---|---|---|
| 11 | 40 | 1,4 | 99,5 |
| 12 | 60 | 1,2 | 98,9 |
| 13 | 80 | 1,8 | 96,5 |

## BEISPIELE 14 BIS 20

Die Arbeitsweise entspricht der des Beispiels 1. Vorgelegt werden nacheinander 0,75 g einer platinhaltigen Aktivkohle (sog. Entfärbekohle mit 1 Gew.-% Platingehalt), eine Lösung von 15,2 g (0,1 Mol) D,L-Mandelsäure in 100 ml Natronlauge verschiedener Normalität und Blei(II)-nitrat in Form einer 0,05-molaren Lösung. Oxidiert wird bei 30°C und einem $O_2$-Druck von 1 bar bis zur Aufnahme von 0,05 Mol $O_2$.

In Tabelle 4 sind die in Abhängigkeit vom Mol-Verhältnis NaOH/Mandelsäure bei verschiedenen Mol-Verhältnissen Blei/Mandelsäure erzielten Ausbeuten und die zu ihrer Erlangung angewandten Reaktionszeiten angegeben:

TABELLE 4

| Beispiel Nr. | Mol-Verhältnis NaOH /Mandelsäure | Mol-Verhältnis Blei $^{2+}$/Mandelsäure | Reaktionsdauer (Stunden) | Phenylglyoxylsäure-Ausbeute (% d. Th.) |
|---|---|---|---|---|
| 14 | 1,5 | 2,5 . $10^{-5}$ | 2 | 99 |
| 15 | 1,5 | 2,5 . $10^{-4}$ | 0,5 | 99 |
| 16 | 1,75 | 2,5 . $10^{-4}$ | 0,5 | 98 |
| 17 | 2,0 | 2,5 . $10^{-4}$ | 0,5 | 94 |
| 18 | 2,5 | 2,5 . $10^{-3}$ | 0,5 | 95 |
| 19 | 3,0 | 2,5 . $10^{-3}$ | 0,5 | 86 |
| 20 | 4,0 | 2,5 . $10^{-3}$ | 0,5 | 84 |

BEISPIEL 21

Die Arbeitsweise entspricht der des Beispiels 1. Vorgelegt werden in der Oxidationapparatur 0,75 g Aktivkohle mit 1 Gew.-% Platingehalt, eine Lösung von 38 g (0,25 Mol) D,L-Mandelsäure in 100 ml 3 n Natronlauge und 1 x $10^{-4}$ Mol $Pb(NO_3)_2$. Oxidiert wird bei 70°C und einem $O_2$-Druck von 1 bar bis zur Aufnahme der stöchiometrisch erforderlichen Sauerstoffmenge (0,125 Mol).

Nach Abtrennen des Katalysators wird dieser erneut für die Oxidation von Mandelsäure eingesetzt, d.h. er wird wieder zu einer Mischung aus 38 g (0,25 Mol) D,L-Mandelsäure, 100 ml 3 n NaOH und 1 x $10^{-4}$ Mol $Pb(NO_3)_2$ gegeben und wie vorstehend oxidiert. Der abgetrennte Katalysator wird erneut für die Oxidation eingesetzt, und so fort. Insgesamt wird der Katalysator 10 mal verwendet. Die Phenylglyoxylsäure-Ausbeuten liegen bei den Versuchen stets zwischen 91—97%. Die pro Mol Mandelsäure eingesetzte Platinmenge ist infolge der Wiederverwendung bereits auf ca. 3 mg gesunken.

Die Versuche wurden bei diesem Ergebnis abgebrochen.

BEISPIEL 22

Es wird wie in Beispiel 1 beschrieben gearbeitet, jedoch mit dem Unterschied, daß die 5 x $10^{-5}$ Mol Blei nicht in Form einer Blei(II)-nitrat-Lösung in die Reaktionsmischung eingebracht werden, sondern in Form feingepulverten

a) Bleimetalls
b) Blei(II)-oxids
c) Blei(II)-sulfates
d) Blei(II)-acetates
e) Blei (II,IV)-oxids (Mennige)
f) Blei (IV)-oxids

zugesetzt werden. Die Reaktionszeit beträgt in allen Fällen wiederum nur 15 Minuten und die Ausbeute stets über 99% d.Th.

BEISPIEL 23

Es wird wie in Beispiel 1 beschrieben gearbeitet, jedoch mit dem Unterschied, daß nicht Blei-, sondern 2 x $10^{-4}$ Mol Wismut in Form seines feingepulverten Nitrats ($Bi(NO_3)_3 . 5 H_2O$) zur Reaktionsmischung gegeben wird. Nach einer Oxidationszeit von 20 Minuten ist die stöchiometrisch erforderliche Sauerstoffmenge aufgenommen. Die polarographische Bestimmung ergibt eine Phenylglyoxylsäure-Ausbeute von 98% d.Th. Der Kontakt kann nach Abfiltrieren wiederverwendet werden. Ohne Wismut-Zusatz wird in dieser Zeit keine Mandelsäure-Oxidation beobachtet.

BEISPIEL 24 BIS 25

Gearbeitet wird wie in Beispiel 1. Oxidiert wird eine Lösung von 38 g (0,25 Mol) D,L-Mandelsäure in 100 ml (0,27 Mol) 10%iger Natronlauge in Gegenwart von 0,75 g Aktivkohle mit 1 Gew.-% Platingehalt bei 70°C mit $O_2$ von 1 bar Druck. Zur Reaktionsmischung wird vor der Oxidation noch $2 \times 10^{-4}$ Mol $Bi(NO_3)_3 . 5 H_2O$ gegeben. Die Oxidation erfordert 1,25 Stunden. Danach sind 0.125 Mol Sauerstoff aufgenommen und die Phenylglyoxylsäure-Ausbeute beträgt 96% d.Th. Wird bei 40°C gearbeitet, so beträgt die Reaktionszeit 4,4 Stunden und die Ausbeute 97% d.Th.

BEISPIEL 26

Es wird wie im Beispiel 1 gearbeitet. Oxidiert wird eine Lösung von 15,2 g (0,1 Mol) D,L-Mandelsäure in 100 ml 1,7 n Natronlauge. Zur Lösung werden 0,75 g Aktivkohle (Medizinalkohle) mit 7,5 Gew.-% Palladiumgehalt und $2,5 \times 10^{-5}$ Mol $Pb(NO_3)_2$ gegeben. Dann wird bei 65°C und 1 bar Sauerstoff-Druck oxidiert. Nach 3,5 Stunden sind 0,05 Mol $O_2$ aufgenommen und die Ausbeute an Phenylgloxylsäure beträgt 46% d.Th.

Ohne Zusatz von Blei ist in dieser Zeit unter sonst gleichen Bedingungen praktisch keine Sauerstoffaufnahme (Oxidation) festzustellen.

BEISPIEL 27

Es wird wie in Beispiel 1 gearbeitet. Oxidiert wird eine Lösung von 15,2 g (0,1 Mol) D,L-Mandelsäure in 100 ml 2,0 n Natronlauge. Zur Lösung werden 1,5 g Aktivkohle (Medicinalkohle) mit 5% Palladiumgehalt sowie $2 \times 10^{-4}$ Mol $Bi(NO_3)_3 . 5 H_2O$ gegeben. Dann wird bei 70°C und 1 bar $O_2$-Druck oxidiert. Nach 10 Minuten sind 0,05 Mol $O_2$ aufgenommen und die Phenylglyoxylsäure-Ausbeute beträgt 50% d.Th.

Ohne Zusatz von Blei wird unter sonst gleichen Bedingungen keine $O_2$-Aufnahme (Oxidation) beobachtet.

BEISPIEL 28

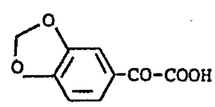

Es wird wie in Beispiel 1 gearbeitet. Zu einer Lösung von 39,2 g (0,2 Mol) 3,4-Methylendioxy-D,L-mandelsäure in 100 ml 2,2 n Natronlauge werden 0,75 g Aktivkohle mit 1 Gew.-% Platingehalt sowie 0,1 g $Bi(NO_3)_3 . 5 H_2O$ ($2 \times 10^{-4}$ Mol) gegeben. Dann wird bei 70°C und 1 bar $O_2$-Druck oxidiert. Nach 2 Stunden sind 0,1 Mol Sauerstoff aufgenommen und die Oxidation kommt zum Stillstand.

Nach Absaugen des Kontaktes wird das Filtrat mit 20%iger Salzsäure auf pH 1 angesäuert, auf ca. 10°C abgeküht, das ausgefallene Produkt wird abgesaugt, mit Eiswasser gewaschen und getrocknet. Erhalten werden 38,2 g einer 98,5%igen 3,4-Methylendioxy-phenylglyoxylsäure, Fp. 144—146°C. Ausbeute: 97% d.Th.

BEISPIEL 29

Zu 0,75 g Aktivkohle mit 1 Gew.-% Platingehalt werden 0,5 ml 0,5-molare $Pb(NO_3)_2$-lösung ($2,5 \times 10^{-3}$ Mol Pb) sowie eine Lösung von 19,6 g (0,1 Mol) 3,4-Methylendioxy-mandelsäure in 100 ml 1,5 n Natronlauge gegeben. Dann wird bei 70°C und 1 bar $O_2$-Druck wie in Beispiel 1 beschrieben oxidiert. Nach 25 Minuten sind 0,05 Mol Sauerstoff aufgenommen und die Oxidation kommt zum Stillstand. Der Kontakt wird abgesaugt, das Filtrat mit 20%iger Salzsäure auf pH 1 angesäuert und mit Eis gekühlt. Die ausgefallene 3,4-Methylendioxy-phenylglyoxylsäure wird abgesaugt, mit Eiswasser gewaschen und getrocknet: 18,2 g (Reinheit ca. 99%), Fp. 144—146°C.

Aus der Mutterlauge können durch Extraktion mit Äther noch ca. 1,5 g der gleichen Säure (Reinheit ca. 80%), Fp. 127—138°, isoliert werden.

Ausbeute insgesamt: 99% d.Th.

BEISPIEL 30

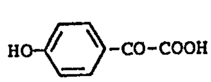

Eine Lösung von 8,4 g (0,05 Mol) 4-Hydroxy-D,L-mandelsäure in 100 ml 2 n Natronlauge wird zu 1 g Aktivkohle mit 1% Platingehalt gegeben und nach Zugabe von $2,5 \times 10^{-3}$ Mol $Pb(NO_3)_2$ bei 30°C und 1 bar Sauerstoffdruck oxidiert. Nach 40 Minuten sind rund 0,021 Mol Sauerstoff aufgenommen. Eine nach Abfiltrieren des Kontaktes durchgeführte polarographische Bestimmung ergibt eine Ausbeute von 83% d.Th. 4-Hydroxy-phenylglyoxylsäure.

BEISPIEL 31

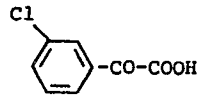

Eine Lösung von 18,6 g (0,1 Mol) 3-Chlor-D,L-mandelsäure in 100 ml 1,5 n Natronlauge wird zu 0,95 g Aktivkohle mit 1% Platingehalt gegeben und nach Zusatz von 2,5 x $10^{-3}$ Mol $Pb(NO_3)_2$ bei 70°C und 1 bar $O_2$-Druck oxidiert. Nach 25 Minuten, wenn 0,05 Mol $O_2$ aufgenommen sind, wird der Kontakt abfiltriert. Eine polarographische Bestimmung ergibt eine 3-Chlor-phenylglyoxylsäure-Ausbeute von 87,9% d.Th.

Aus der Lösung können durch Ansäuern mit 20%iger Salzsäure, Abkühlen mit Eiswasser, Absaugen und Trocknen 16,2 g 3-Chlor-phenylglyoxylsäure, Fp. 59—62°C, isoliert werden.

BEISPIEL 32

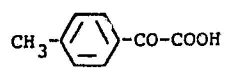

Es wird wie in Beispiel 1 gearbeitet, jedoch mit dem Unterschied, daß 8,3 g (0,05 Mol) 4-Methyl-D,L-mandelsäure in 50 ml 1,5 n Natronlauge gelöst und in Gegenwart von 0,4 g Aktivkohle mit 1 Gew.-% Platingehalt und 0,25 ml 0,5-molarer Blei(II)-nitrat-lösung ≙ 1,25 x $10^{-4}$ Mol Pb) bei 70°C und guter Durchmischung bei 1 bar $O_2$-Druck oxidiert werden. Nach 40 Minuten sind 0,025 Mol Sauerstoff aufgenommen und die Oxidation kommt zum Stillstand. Nach Abfiltrieren des Kontaktes ergibt die polarographische Bestimmung gegen reine p-Tolylgloxylsäure eine Ausbeute von 93% d.Th.

Die p-Tolylglyoxylsäure fällt beim Ansäuern auf pH 1 zunächst als Öl aus, das mit Äther ausgeschüttelt werden kann. Nach Abdampfen des Äthers verbleiben ca. 7,5 g p-Tolylglyoxylsäure vom Fp. 84—86°C, woraus durch Sublimation im Vakuum reine p-Tolylglyoxylsäure vom Fp. 98—99°C erhalten werden kann.

BEISPIEL 33

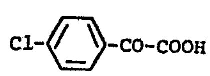

Es wird wie in Beispiel 1 gearbeitet, jedoch mit dem Unterschied, daß 18,6 g (0,1 Mol) 4-Chlor-D,L-mandelsäure in 100 ml 1,35 n Natronlauge gelöst und nach Zugabe von 1 g Aktivkohle mit 1 Gew.-% Platin-Gehalt und 0,5 ml 0,5-molarer $Pb(NO_3)_2$-Lösung ≙ 2,5 x $10^{-4}$ Mol Blei) bei 70°C und 1 bar $O_2$-Druck unter guter Durchmischung oxidiert werden. Nach 60 Minuten sind 0,05 Mol $O_2$ aufgenommen, und nach dem Abfiltrieren des Kontaktes ergibt die polarographische Bestimmung gegen reine 4-Chlor-phenylglyoxylsäure als internen Standard eine Ausbeute an 4-Chlor-phenylglyoxylsäure von 86,5% d.Th.

Die 4-Chlor-phenylglyoxylsäure kann durch Ansäuern mit HCl ausgefällt und aus Wasser umkristallisiert werden: Fp. 61—62°C. Bei Umkristallisation aus Ligroin erhält man nach scharfem Trocknen die wasserfreie Form mit einem Fp. von 91—92°C.

**Patentansprüche**

1. Verfahren zur Herstellung von Arylglyoxylsäuren durch Oxidation von $\alpha$-Hydroxyarylessigsäuren der Formel

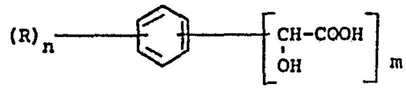
(I)

in der

m für 1, 2 oder 3 steht,

n für die Zahl, die sich aus der Differenz (6-m) ergibt, steht und die Reste

R entweder einzeln und unabhängig voneinander für Wasserstoff, Alkyl, Cycloalkyl, Aryl, Aralkyl, Alkoxy, Cycloalkoxy, Aryloxy, Hydroxy, Halogen, Aminoalkyl und/oder Carboxy stehen, oder in der Zwei Reste R gemeinsam für die Methylendioxygruppe oder einen anderen anellierten, gegebenenfalls heterocyclischen und gegebenenfalls substituierten Ring stehen,

dadurch gekennzeichnet, daß man die Oxidation mit Sauerstoff oder molekularen Sauerstoff enthaltenden Gasen in wäßrigalkalischem Medium in Gegenwart von Platinmetall-Katalysatoren bei gleichzeitiger Anwesenheit von Blei und/oder Wismut und/oder deren Verbindungen und gegebenenfalls in Gegenwart eines inerten organischen Lösungsmittels bei Temperaturen bis zum Siedepunkt des Reaktionsgemisches durchführt.

2. Verfahren gemäß Anspruch 1, dadurch gekennzeichnet, daß man Blei und/oder Wismut in Mengen von $5 \times 10^{-6}$ bis $1 \times 10^{-1}$ Mol je Mol zu oxidierende $\alpha$-Hydroxyessigsäuregruppe einsetzt.

3. Verfahren gemäß Anspruch 1, dadurch gekennzeichnet, daß man Blei und/oder Wismut in Mengen von $1 \times 10^{-5}$ bis $1 \times 10^{-2}$ Mol je Mol zu oxidierende $\alpha$-Hydroxyessigsäuregruppe einsetzt.

4. Verfahren gemäß Anspruch 1, dadurch gekennzeichnet, daß man als Platinmetall-Katalysatoren Platin und/oder Palladium verwendet.

5. Verfahren gemäß Anspruch 1, dadurch gekennzeichnet, daß man Blei und/oder Wismut in Kombination mit Platinkatalysatoren verwendet.

6. Verfahren gemäß Anspruch 1, dadurch gekennzeichnet, daß man als Träger für das Platinmetall-Aktivkohle verwendet.

7. Verfahren gemäß Anspruch 1, dadurch gekennzeichnet, daß der Platinmetallgehalt der Trägerkatalysatoren unter 10 Gew.-% liegt.

8. Verfahren gemäß Anspruch 1, dadurch gekennzeichnet, daß als Alkali Natrium- oder Kaliumhydroxid in Mengen von 0,3 bis 5 Mol, vorzugsweise von 0,5 bis 2,5 Mol, insbesondere von 0,9 bis 1,8 Mol pro Mol Carboxylgruppe (in der zu oxidierenden $\alpha$-Hydroxyessigsäure (I)) eingesetzt werden.

9. Verfahren gemäß Anspruch 1, dadurch gekennzeichnet, daß man bei Temperaturen von 0° bis 100°C arbeitet.

10. Verfahren gemäß Anspruch 1, dadurch gekennzeichnet, daß man mit Sauerstoff oder Sauerstoff enthaltenden Gasen bei einem Druck von 0,5—10 bar arbeitet.

## Claims

1. Process for the preparation of arylglyoxylic acids by oxidation of $\alpha$-hydroxyarylacetic acids of the formula

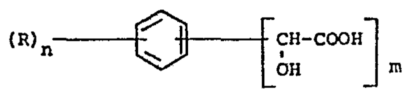

(I)

in which

m represents 1, 2 or 3,

n represents the number obtained from the difference (6-m) and

the radicals R either individually and independently of one another represent hydrogen, alkyl, cycloalkyl, aryl, aralkyl, alkoxy, cycloalkoxy, aryloxy, hydroxyl, halogen, aminoalkyl and/or carboxyl, or

in which

two radicals R together represent the methylenedioxy group or another fused-on, optionally heterocyclic and optionally substituted ring,

characterised in that the oxidation is carried out with oxygen or gases containing molecular oxygen, in an aqueous-alkaline medium in the presence of platinum metal catalysts, lead and/or bismuth and/or compounds thereof simultaneously being present, and if appropriate in the presence of an inert organic solvent, at temperatures up to the boiling point of the reaction mixture.

2. Process according to Claim 1, characterised in that lead and/or bismuth are employed in amounts of $5 \times 10^{-6}$ to $1 \times 10^{-1}$ mol per mol of $\alpha$-hydroxyacetic acid group to be oxidised.

3. Process according to Claim 1, characterised in that lead and/or bismuth are employed in amounts of $1 \times 10^{-5}$ to $1 \times 10^{-2}$ mol per mol of $\alpha$-hydroxyacetic acid group to be oxidised.

4. Process according to Claim 1, characterised in that platinum and/or palladium are used as the platinum metal catalysts.

5. Process according to Claim 1, characterised in that lead and/or bismuth are used in combination with platinum catalysts.

6. Process according to Claim 1, characterised in that active charcoal is used as a support for the platinum metal.

7. Process according to Claim 1, characterised in that the platinum metal content of the supported catalysts is less than 10% by weight.

8. Process according to Claim 1, characterised in that sodium hydroxide or potassium hydroxide is employed as the alkali, in amounts of 0.3 to 5 mols, preferably of 0.5 to 2.5 mols and in particular of 0.9 to 1.8 mols, per mol of carboxyl group (in the $\alpha$-hydroxyacetic acid (I) to be oxidised).

9. Process according to Claim 1, characterised in that it is carried out at temperatures from 0° to 100°C.

10. Process according to Claim 1, characterised in that it is carried out with oxygen or oxygen-containing gases under a pressure of 0.5—10 bars.

## Revendications

1. Procédé de préparation d'acides arylglyoxyliques par oxydation d'acides alpha-hydroxyarylacétiques répondant à la formule

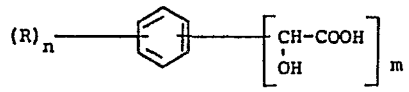

(I) ·

dans laquelle
m est égal à 1, 2 ou 3,
n est un nombre égal à la différence 6-m, et
les symboles R représentent chacun, indépendamment les uns des autres, un atome d'hydrogène, un groupe alkyle, cycloalkyle, aryle, aralkyle, alcoxy, cycloalcoxy, aryloxy, hydroxy, un atome d'halogène, un groupe aminoalkyle et/ou carboxy, ou bien deux symboles R représentent ensemble le groupe méthylène-dioxy ou un autre cycle condensé, éventuellement hétérocyclique et éventuellement substitué,
ce procédé se caractérisant en ce que l'on effectue l'oxydation à l'aide d'oxygène ou de gaz contenant de l'oxygène moléculaire en milieu aqueux alcalin en présence de catalyseurs à base de métaux de la série du platine et en présence simultanée de plomb et/ou de bismuth et/ou de leurs composés et, le cas échéant, en présence d'un solvant organique inerte, à des températures allant jusqu'au point d'ébullition du mélange de réaction.

2. Procédé selon la revendication 1, caractérisé en ce que l'on utilise le plomb et/ou le bismuth en quantité de $5 \times 10^{-6}$ à $1 \times 10^{-1}$ mole par mole de groupe acide alpha-hydroxyacétique à oxyder.

3. Procédé selon la revendication 1, caractérisé en ce que l'on utilise le plomb et/ou bismuth en quantité de $10^{-5}$ à $10^{-2}$ mole par mole de groupe acide alpha-hydroxyacétique à oxyder.

4. Procédé selon la revendication 1, caractérisé en ce que l'on utilise en tant que metaux catalyseurs de la série du platine le platine et/ou le palladium.

5. Procédé selon la revendication 1, caractérisé en ce que l'on utilise le plomb et/ou le bismuth en combinaison avec des catalyseurs au platine.

6. Procédé selon la revendication 1, caractérisé en ce que l'on utilise le carbon actif en tant que support du métal de la série du platine.

7. Procédé selon la revendication 1, caractérisé en ce que la teneur en métal de la série du platine des catalyseurs sur support est inférieure à 10% en poids.

8. Procédé selon la revendication 1, caractérisé en ce que l'on utilise en tant qu'alcali l'hydroxyde de sodium ou de potassium en quantité de 0,3 à 5 moles, de préférence de 0,5 à 2,5 moles, plus spécialement de 0,9 à 1,8 mole par mole de groupe carboxyle (contenu dans l'acide alpha-hydroxyacétique I à oxyder).

9. Procédé selon la revendication 1, caractérisé en ce que l'on opère à des températures de 0 à 100°C.

10. Procédé selon la revendication 1, caractérisé en ce que l'on opère avec l'oxygène ou des gaz contenant de l'oxygène sous une pression de 0,5 à 10 bars.